Europäisches Patentamt

European Patent Office

Office européen des brevets

(11) Publication number: **0 299 720 B1**

# EUROPEAN PATENT SPECIFICATION

(45) Date of publication of patent specification: **29.09.93**    (51) Int. Cl.⁵: **C07C  31/125**, C07C 29/34

(21) Application number: **88306359.6**

(22) Date of filing: **12.07.88**

(54) Process for the condensation of alcohols.

(30) Priority: **13.07.87 US 72633**

(43) Date of publication of application:
**18.01.89 Bulletin  89/03**

(45) Publication of the grant of the patent:
**29.09.93 Bulletin  93/39**

(84) Designated Contracting States:
**AT BE CH DE ES FR GB IT LI LU NL SE**

(56) References cited:
**US-A- 3 514 493**

(73) Proprietor: **EXXON CHEMICAL PATENTS INC.**
**200 Park Avenue**
**Florham Park New Jersey 07932(US)**

(72) Inventor: **Pruett, Roy Lavelle**
**8431 Willow Oak Lane**
**Harrisburg North Carolina 28075(US)**
Inventor: **Young, David Alexander**
**1621 Sherwood Forest Boulevard**
**Baton Rouge Louisiana 70815(US)**
Inventor: **Duncan, Carolyn Boggus**
**17722 Sharpsburg Avenue**
**Baton Rouge Louisiana 70817(US)**
Inventor: **Mozeleski, Edmund John**
**267 RD 3, Hoffman Drive**
**Califon New Jersey 07830(US)**

(74) Representative: **Bawden, Peter Charles et al**
**EXXON CHEMICAL LIMITED EXXON CHEMI-**
**CAL TECHNOLOGY CENTRE PO Box 1**
**Abingdon Oxfordshire OX13 6BB (GB)**

# EP 0 299 720 B1

**Description**

This invention relates to a process for preparing a Guerbet alcohol, which is a branched dimerized or condensed alcohol resulting from the condensation of a primary alcohol or a mixture of primary alcohols. More particularly, this invention relates to an improved Guerbet process characterized in the use of very small proportions of platinum catalyst which are capable of providing high conversions to the condensed alcohol product.

The Guerbet reaction for condensing primary alcohols to provide a branched condensed alcohol product is well known in the art and the use of noble metal catalysts in combination with alkali metal hydroxides has been generally disclosed.

U.S. Patent 2,457,866 discloses the use of alkali condensation catalysts and a small amount of a dehydrogenation catalyst with rapid removal of water as it is formed in the course of the reaction.

U.S. Patent 2,762,847 discloses the use of phosphates as catalysts for the Guerbet reaction, particularly potassium phosphates.

U.S. Patent 2,862,013 discloses high molecular weight, branched chain alcohols having 24 to 28 carbon atoms which are prepared by Guerbet condensation using generally any inorganic base and any known dehydrogenation catalyst.

U.S. Patent 3,119,880 discloses a catalyst for the Guerbet condensation reaction which is a combination of an alkali and a lead salt, preferably with another catalyst, which can be any dehydrogenation catalyst.

U.S. Patent 3,514,493 discloses a process for the dimerization of non-branched primary alcohols, secondary alcohols, or mixtures of non-branched primary alcohols with primary alcohols branched in the 2-position. The catalyst is an alkaline condensing agent and a metal which may be platinum, palladium, ruthenium or rhodium, either supported or unsupported. The reaction is conducted at 80°C to 300°C, preferably at temperatures less than 160°C. The noble metal catalysts may be used on a number of supports, including activated carbon as a preferred support. The amount of noble metal used is disclosed as 0.0001 to 10 parts by weight, preferably from 0.002 to 1 part by weight per 100 parts of alcohol. The alkaline condensing agent is used in amounts of from 0.1 to 50 mols, preferably 2 to 10 mols per 100 mols of alcohol.

U.S. Patent 3,860,664 discloses an improvement in the Guerbet reaction rate by carrying out the reaction in the presence of an alkali metal catalyst and certain palladium compounds, provided that the palladium compound is introduced to the alkanol reactant prior to dissolution of the alkali metal catalyst.

U.S. Patent 4,518,810 discloses a Guerbet process for preparing a branched dimerized alcohol using a copper-nickel catalyst in combination with an alkaline substance.

Of these references, U.S. 3,514,493 is considered the most pertinent since it contains a general disclosure of the use of metals including platinum in combination with an alkali metal condensing agent. However, the inventors hereof have found that, when using platinum in combination with KOH or NaOH, almost no measurable conversion to dimerized alcohol is obtained using the very low levels said to be operable in U.S. Patent 3,514,593. The present invention is based on the discovery that there is an important relationship among platinum concentration, KOH or NaOH concentration and reaction temperature which permits a high level of conversion to dimeric alcohols of about 85% or greater over reaction times of about 1 to 5 hours with minimization of formation of unwanted by-products. In particular, under the conditions of the present invention, such high conversions may be achieved in reaction times substantially less than those disclosed in the prior art as represented by U.s. Patent 3,514,493.

In accordance with the present invention there has been discovered a process for preparing a branched dimer alcohol which comprises heating and condensing a primary alcohol of the formula $RCH_2CH_2OH$ wherein R is an alkyl having 1 to 20 carbon atoms or a mixture of such alcohols at a temperature of 175°C to 210°C in the presence of a catalyst system consisting essentially of platinum on an activated carbon support and potassium or sodium hydroxide, the amount of platinum being at least 0.0008 wt. % up to 0.0032 wt. % and the amount of potassium or sodium hydroxide being from 1.4 wt. % to 6.8. wt. %, said percentages being based upon the total weight of alcohols in the reaction mixture, said process providing a conversion of said primary alcohol to branched dimer alcohol of at least 85% in a reaction time of 1 to 5 hours or less.

Preferably, to achieve maximum conversions in excess of 85% at reaction times of 1 to 2 hours at a temperature range of 175°C to 190°C, there is employed at least 0.0016 wt. % platinum and at least 2.8 wt. % potassium or sodium hydroxide. At a temperature range of 190°C to 210°C, there is employed at least 0.0008 wt.% platinum and at least 1.4 wt. % potassium or sodium hydroxide to achieve maximum conversions of 85% or more at reaction times of 1 to 2 hours.

2

The optimum conditions for practicing the process of the present invention have been found to be a temperature of 183°C, a platinum concentration of 0.0024 wt. %, a potassium hydroxide or sodium hydroxide concentration of 5.1 wt. %, with potassium hydroxide being preferred. Under these conditions essentially complete conversion, i.e., on the order of 95% or greater, will be achieved in reaction times of 1 hour.

The present invention applies generally to the dimerization of alcohols or mixtures of alcohols of the formula $RCH_2CH_2OH$ when R is an alkyl or 1 to 20 carbon atoms. The alkyl group may be a straight or branched chain alkyl group. Preferred for use in the process of the present invention are $C_6$ to $C_{13}$ oxo alcohols which are those alcohols produced by the hydroformylation of olefins and which are mixtures of branched chain primary alcohols such as branched chain oxo octyl alcohols or branched chain decyl alcohols. These alcohols normally contain about 20% by weight of alcohols having branching at the 2-carbon position and these alcohols cannot be condensed in the Guerbet reaction. Generally, these oxo alcohols are mostly methyl branched with about 1 to 5 branches per molecule.

The present invention is also applicable to the Guerbet condensation of a mixture of alcohols having different numbers of carbon atoms. Thus, a mixture of $C_8$ to $C_{10}$ oxo alcohols will provide a mixture of products consisting of $C_{16}$, $C_{18}$ and $C_{20}$ alcohols resulting from the condensation of the alcohols. Similarly, n-butanol and decyl alcohol may be condensed in accordance with the invention to produce a mixture of $C_{20}$ alcohol, $C_{14}$ alcohol (a 2-substituted butyl decanol) and 2-ethyl hexanol.

The general procedure for conducting the process of the invention comprises the steps of first combining the alcohol reaction mixture and the KOH or NaOH and heating this mixture until the base dissolves. Thereafter the platinum catalyst on activated carbon support is added, the reaction mixture is heated to reflux with water being evolved and removed as it is formed, which is a standard procedure in the Guerbet condensation of alcohols. A preferred method in the present invention comprises including in the alcohol reaction mixture 10 to 20 wt. % of a non-reactive 2-substituted lower alcohol having up to about 12 carbon atoms, such as 2-ethyl hexanol which has been found particularly effective in promoting and maintaining reflux conditions and removing water azeotropically. Alternate techniques known in the art for removing water may also be used, such as nitrogen stripping. After completion of the reaction, i.e., when water evolution has ceased or when the desired reaction time is reached, the solution is cooled, water and a low molecular weight carboxylic acid, such as formic or acetic acid, is added and the alkali metal is extracted as an acetate salt. The organic layer is separated and filtered to remove the platinum catalyst.

The platinum employed in the process of this invention is used as platinum metal dispersed onto a high surface area activated carbon support, preferably a 5 wt. % platinum on carbon catalyst is used, but the amount of platinum dispersed onto the activated carbon may vary from about 1 to 10 wt. % based on the total weight of platinum and activated carbon. The platinum/activated carbon catalyst should have a surface area of at least 500 $M^2$/g. (square meters per gram) and a particle size of less than 200 microns. Preferably the platinum/activated carbon catalyst will have a surface area of 1000 to 1200 $M^2$/g. and a mean particle size of less than 100 microns, such as about 30 microns with 90% by weight of the particles having a particle size less than 70 microns.

A particular advantage of the present invention is that the amount of platinum required is the minimum amount required to achieve very high levels of conversion. This results in a substantial economic advantage because of the high cost of platinum. Amounts of platinum in excess of the ranges disclosed herein are unnecessary in order to achieve conversions on the order of 85% on higher. The amounts of platinum used herein do represent an advance in the art. For example, in U.S. Patent 3,514,493, Example 3, there was used 500 mg of carbon containing 10% of platinum to react 158.5 g. of butanol. This amounts to 0.03 wt. % platinum. However, after 20 hours, the conversion was only 45%. The present invention uses significantly less platinum and obtains conversions of 85% or more in reaction times of less than 5 hours.

It has been discovered that when the foregoing conditions are met consistently high conversions of alcohols to condensed or dimerized product are obtained with minimization or elimination of unwanted carbonyl-containing by-products.

The invention is further illustrated by the following examples which are not to be considered as limitative of its scope.

In each of the examples below a three-necked round bottom flask was fitted with a Dean-Stark trap for collecting evolved water, a reflux condenser, thermometer, a vacuum source and a magnetic stirrer. Alcohol or alcohols and a base were added, with warming until the base dissolved. The solution was heated to about 178°C until water ceased being evolved. The solution was then cooled, the platinum catalyst was added and the reaction mixture was heated to the desired reaction temperature with sufficient vacuum to cause reflux and entrapment of the evolved water; the pressure was decreased to maintain reflux at the required temperature during the course of the reaction.

3

In all examples, the catalyst used was 5% platinum dispersed onto activated carbon powder having a surface area of 1200 $M^2$g. and a mean particle size of 30 microns with 90% of the powder having a particle size less than 70 microns.

Examples 1-5

In these examples the alcohol used was 210 g. 1-decanol with 40 g. of 2-ethyl-1-hexanol added to maintain reflux conditions. The reaction time was one hour. Percentages of KOH and 5% Pt on activated carbon are based on the total amount of 1-decanol and 2-ethyl-1-hexanol in the reaction mixture.

| Example | % KOH | % Pt/C | T°C | % Conversion |
|---------|-------|--------|-----|--------------|
| 1 | 6.76 | 0.0032 | 190 | 99.6 |
| 2 | 6.76 | 0.0032 | 175 | 90.9 |
| 3 | 6.76 | 0.0016 | 190 | 87.9 |
| 4 | 3.40 | 0.0032 | 190 | 86.8 |
| 5 | 5.08 | 0.0024 | 183 | 97.1 |

Comparative Examples - One Hour Reaction Time

A - Example 5 was repeated at 158°C and a conversion of 24.5% was obtained.
B - Example 4 was repeated at 175°C and a conversion of 40.3% was obtained. Longer reaction times will increase the conversion to 85%.
In the following Examples 6-10, alcohols were used which comprised oxo alcohols having predominantly methyl branching with about 20% of the branching being at the 2-carbon position. Such alcohols having substituents on the 2-carbon position are unreactive in the Guerbet condensation process. Thus, conversions on these examples are reported as percent conversions of convertible alcohols which means the quantity of alcohols present which are capable of undergoing reaction to form a Guerbet condensed or dimerized alcohol product.

Example 6

The reactant alcohol was an isodecyl alcohol commercial product made by hydroformylation of branched nonenes and known as oxo alcohol. This alcohol, 250 g. was subjected to the conditions of the foregoing examples. After one hour, 7.8 ml. of $H_2O$ had been evolved and 78% of the convertible alcohol had been converted.

Example 7

Commercial isodecyl alcohol was subjected to the conditions of the preceding examples. After 50 minutes, 7.0 ml. of water was evolved and no further was evolved in the next 10 minutes. The product analyzed for 82% conversion of the convertible alcohol.

Example 8

Commercial isooctyl alcohol, manufactured by hydroformylation of mixed heptenes, was subjected to the reaction conditions of Example 12. After 110 minutes, evolution of water was complete, 11.3 ml. Analysis of the product showed 90% conversion of the convertible alcohols.

Example 9

Commercial isotridecyl alcohol, manufactured by hydroformylation of mixed dodecenes, was subjected to the reaction conditions of the preceding examples. After 100 minutes, evolution of water was complete, 6.3 ml. Analysis of the product by gas chromatography showed 86% conversion of the convertible alcohol.

4

Example 10

An equimolar mixture of isooctyl alcohol (97.7 g.) and isodecyl alcohol (118.7 g.) was subjected to the reaction conditions of Example 12. After 1.25 hours at temperature, 8.7 ml. of water had been evolved and evolution had ceased. Heating was continued for another 15 minutes to ensure completion. Analysis of the product indicated 86% conversion (of convertible alcohols) to a product mixture of $C_{16}$, $C_{18}$, $C_{20}$ alcohols in an approximate 1:2:1 ratio. This example indicates that the process of this invention may be used to prepare a range of carbon-number alcohols by use of mixed alcohols as starting material.

The following Examples 11-18, demonstrate the applicability of the process of the present invention to the reaction of mixed alcohols. In these examples mixtures of oxo isodecyl alcohol and n-butanol were reacted. The product mix which is produced comprises dimer $C_{20}$ alcohol, chain-extended $C_{14}$ alcohol and 2-ethyl hexanol. The procedure for reactions was the same as the preceding examples. The maximum conversion that can be expected is a function of the contribution of the branched oxo isodecyl alcohol component, 80% of which is convertible, since 20% of this alcohol has branching at the 2 carbon position. In these examples the amount of KOH was varied as indicated in the Table but 0.16 g. of 5% Pt on activated carbon was used (1200 $M^2$/g. particles with mean particle size of 30 microns and 90% of particles being less than 70 microns). In each of Examples 11-18, the amount of unreacted n-butanol was very small, varying from 2.9% to 4.9%.

5

## Table – Examples 11-18

| Example | C10, g. | Added C4, g. | KOH, g. | Time, Min. | T, °C. | H2O, Ml. | C8, % | C10, % | C14, % | C20, % | % C10 Conversion |
|---|---|---|---|---|---|---|---|---|---|---|---|
| 11 | 277 | 74 | 20.0 | 90 | 190 | 13.2 | 6.3 | 26.9 | 31.0 | 30.4 | 83 |
| 12 | 158 | 80 | 10.0 | 180 | 180 | 9.1 | 9.4 | 14.9 | 38.2 | 28.0 | 99 |
| 13 | 158 | 96 | 20.0 | 210 | 190 | 16.4 | 13.3 | 8.8 | 42.4 | 20.7 | 107 |
| 14 | 277 | 68 | 10.0 | 150 | 190 | 16.1 | 4.4 | 19.3 | 28.4 | 38.1 | 95 |
| 15 | 158 | 64 | 10.0 | 120 | 190 | 10.9 | 9.2 | 14.4 | 34.9 | 32.3 | 91 |
| 16 | 277 | 74 | 20.0 | 124 | 180 | 12.6 | 4.7 | 29.7 | 29.4 | 28.0 | 78 |
| 17 | 158 | 130 | 20.0 | 340 | 180 | 13.0 | 16.1 | 9.8 | 42.6 | 17.4 | 104 |
| 18 | 277 | 67 | 10.0 | 210 | 180 | 14.2 | 4.3 | 30.2 | 29.2 | 27.6 | 77 |

## Notes

$C_{10}$ refers to isodecyl alcohol; $C_4$ refers to n-butanol. The conversion of $C_{10}$ alcohol is based on the maximum convertible amount being 80%. To illustrate, in Example 14 28.4% of the product is $C_{14}$ alcohol but the $C_{10}$ converted is the sum of that which is converted to $C_{14}$, 21.0%, plus that which is converted to $C_{20}$ alcohol, 39.1%, which equals 60.1% of the product. Unconverted $C_{10}$ is 19.3% of the product. Thus the conversion is 60.1/60.1 + 19.3 or 75.7% and the conversion of the convertible alcohols is 75.7/.8 = 95% as reported in the final column of the table. Values in excess of 100% indicate that some 2-branched alcohols have reacted with n-butanol to give $C_{14}$ alcohols.

## Example 19

Following the procedure of Examples 11-18, 200.3 g. of oxo isotridecyl alcohol was treated with 108.2 g. n-butanol over a period of 315 minutes and 12.0 ml. g. water was evolved. The product contained 12.2% $C_8$

EP 0 299 720 B1

alcohol, 14.7% unreacted $C_{13}$ alcohol, 40.5% $C_{17}$ alcohol and 16.0% $C_{26}$ alcohol. The conversion of convertible $C_{13}$ alcohol was 96%.

## Claims

1. A process for preparing a branched alcohol which comprises heating and condensing a primary alcohol of the formula $RCH_2CH_2OH$ or a mixture of said primary alcohols, where R is straight or branched chain alkyl of 1 to 20 carbon atoms, at a temperature of 175°C to 210°C in the presence of a catalyst system consisting of (a) 1-10 wt. % platinum on a high surface area activated carbon support and (b) potassium or sodium hydroxide, the amount of platinum being from 0.0008 wt. % to 0.0032 wt. % and the amount of potassium or sodium hydroxide being from 1.4 wt. % to 6.8%, said percentages being based upon the total weight of alcohols in the reaction mixture.

2. The process of claim 1 in which the heating and condensing is carried out for a period of 1 to 5 hours.

3. The process of claim 1 or claim 2 wherein the catalyst is 5 wt. % platinum on an activated carbon powder having a surface area of at least 500 $M^2$/g and a particle size of less than 200 microns.

4. The process of any of the preceding claims wherein the catalyst is 5 wt. % platinum on an activated carbon powder having a surface area of 1000 to 1200 $M^2$/g. and a mean particle size of 30 microns with at least 90% of the particles being less than 70 microns.

5. The process of any of the preceding claims wherein the activated carbon has a surface area of at least 500 $m^2$/g. and a particle size of less than 200 microns.

6. The process of any of the preceding claims wherein the activated carbon has a surface area of 1000 to 1200 $M^2$/g. and a mean particle size of 30 microns with at least 90% of the particles being less than 70 microns.

7. The process of claims 3 or 4 where the alcohol is a mixture of $C_6$ to $C_{13}$ oxo alcohols having about 20% branching in the 2 carbon position and about 1-5 branches per molecule.

8. The process of claim 3 or 4 wherein the temperature range is 175°C to 190°C, the amount of platinum is at least 0.0016 wt. % and the amount of potassium or sodium hydroxide is at least 2.8wt. %.

9. The process of claims 3 or 4 wherein the temperature range is 190°C to 210°C, the amount of platinum is at least 0.0008 wt. % and the amount of potassium or sodium hydroxide is at least 1.4 wt.%.

10. The process of claims 3 or 4 wherein the temperature is about 183°C, the amount of platinum is about 0.0024 wt. % and the amount of potassium hydroxide or sodium hydroxide is about 5.1 wt. %.

11. The process of claim 10 wherein there is employed potassium hydroxide.

## Patentansprüche

1. Verfahren zur Herstellung eines verzweigten Alkohols, bei dem ein primärer Alkohol mit der Formel $RCH_2CH_2OH$ oder eine Mischung aus solchen primären Alkoholen, wobei R eine geradkettige oder verzweigtkettige Alkylgruppe mit 1 bis 20 Kohlenstoffatomen ist, in Gegenwart eines Katalysatorsystems, das aus (a) 1 bis 10 Gew.% Platin auf einem Träger aus Aktivkohle mit einer hohen Oberfläche und (b) Kalium- oder Natriumhydroxid besteht, wobei die Menge an Platin 0,0008 Gew.% bis 0,0032 Gew.% beträgt und die Menge an Kalium- oder Natriumhydroxid 1,4 Gew.% bis 6,8 Gew.% beträgt, wobei diese Prozentsätze sich auf das Gesamtgewicht der Alkohole in der Reaktionsmischung beziehen, auf eine Temperatur von 175°C bis 210°C erhitzt und kondensiert wird.

2. Verfahren nach Anspruch 1, bei dem das Erhitzen und Kondensieren während einer Zeitdauer von 1 bis 5 Stunden durchgeführt wird.

7

**3.** Verfahren nach Anspruch 1 oder Anspruch 2, bei dem der Katalysator 5 Gew.% Platin auf Aktivkohlepulver mit einer Oberfläche von mindestens 500 $m^2/g$ und einer Teilchengröße von weniger als 200 $\mu m$ ist.

**4.** Verfahren einem der vorhergehenden Ansprüche, bei dem der Katalysator 5 Gew.% Platin auf Aktivkohlepulver mit einer Oberfläche von 1 000 bis 1 200 $m^2/g$ und einer mittleren Teilchengröße von 30 $\mu m$ ist, wobei mindestens 90 % der Teilchen kleiner als 70 $\mu m$ sind.

**5.** Verfahren nach einem der vorhergehenden Ansprüche, bei dem die Aktivkohle eine Oberfläche von mindestens 500 $m^2/g$ und eine Teilchengröße von weniger als 200 $\mu m$ aufweist.

**6.** Verfahren nach einem der vorhergehenden Ansprüche, bei dem die Aktivkohle eine Oberfläche von 1 000 bis 1 200 $m^2/g$ und eine mittlere Teilchengröße von 30 $\mu m$ aufweist, wobei mindestens 90 % der Teilchen kleiner als 70 $\mu m$ sind.

**7.** Verfahren nach Anspruch 3 oder 4, bei dem der Alkohol eine Mischung aus $C_6$- bis $C_{13}$-Oxoalkoholen mit etwa 20 % Verzweigung in der Stellung des 2-Kohlenstoffs und etwa 1 bis 5 Verzweigungen pro Molekül ist.

**8.** Verfahren nach Anspruch 3 oder 4, bei dem der Temperaturbereich 175°C bis 190°C ist, die Menge an Platin mindestens 0,0016 Gew.% beträgt und die Menge an Kalium- oder Natriumhydroxid mindestens 2,8 Gew.% beträgt.

**9.** Verfahren nach Anspruch 3 oder 4, bei dem der Temperaturbereich 190°C bis 210°C ist, die Menge an Platin mindestens 0,0008 Gew.% beträgt und die Menge an Kalium- oder Natriumhydroxid mindestens 1,4 Gew.% beträgt.

**10.** Verfahren nach Anspruch 3 oder 4, bei dem die Temperatur etwa 183°C ist, die Menge an Platin etwa 0,0024 Gew.% beträgt und die Menge an Kalium- oder Natriumhydroxid etwa 5,1 Gew.% beträgt.

**11.** Verfahren nach Anspruch 10, bei dem Kaliumhydroxid verwendet wird.

**Revendications**

**1.** Procédé de production d'un alcool ramifié, qui comprend le chauffage et la condensation d'un alcool primaire de formule $RCH_2CH_2OH$ ou d'un mélange de ces alcools primaires, formule dans laquelle R est un groupe alkyle à chaîne droite ou ramifiée ayant 1 à 20 atomes de carbone, à une température de 175 à 210°C en présence d'une composition de catalyseur constituée (a) de 1 à 10 % en poids de platine sur un support de carbone activé de grande surface spécifique et (b) d'hydroxyde de potassium ou de sodium, la quantité de platine allant de 0,0008 % en poids à 0,0032 % en poids et la quantité d'hydroxyde de potassium ou de sodium allant de 1,4 % en poids à 6,8 % en poids, lesdits pourcentages étant basés sur le poids total d'alcools dans le mélange réactionnel.

**2.** Procédé suivant la revendication 1, dans lequel le chauffage et la condensation sont conduits pendant une période de 1 à 5 heures.

**3.** Procédé suivant la revendication 1 ou la revendication 2, dans lequel le catalyseur consiste en platine à 5 % en poids fixé sur du carbone activé en poudre ayant une surface spécifique d'au moins 500 $m^2/g$ et un diamètre des particules de moins de 200 micromètres.

**4.** Procédé suivant l'une quelconque des revendications précédentes, dans lequel le catalyseur consiste en 5 % en poids de platine sur du carbone activé en poudre ayant une surface spécifique de 1000 à 1200 $m^2/g$ et un diamètre moyen des particules de 30 micromètres, au moins 90 % des particules ayant un diamètre inférieur à 70 micromètres.

**5.** Procédé suivant l'une quelconque des revendications précédentes, dans lequel le carbone activé a une surface spécifique d'au moins 500 $m^2/g$ et un diamètre des particules de moins de 200 micromètres.

**6.** Procédé suivant l'une quelconque des revendications précédentes, dans lequel le carbone activé a une surface spécifique de 1000 à 1200 $m^2$/g et un diamètre moyen des particules de 30 micromètres, au moins 90 % des particules ayant un diamètre inférieur à 70 micromètres.

**7.** Procédé suivant la revendication 3 ou 4, dans lequel l'alcool est un mélange d'oxo-alcools en $C_6$ à $C_{13}$ ayant environ 20 % de ramification sur l'atome de carbone en position 2 et environ 1 à 5 ramifications par molécule.

**8.** Procédé suivant la revendication 3 ou 4, dans lequel la plage de températures va de 175 à 190°C, la quantité de platine est d'au moins 0,0016 % en poids et la quantité d'hydroxyde de potassium ou de sodium est d'au moins 2,8 % en poids.

**9.** Procédé suivant la revendication 3 ou 4, dans lequel la plage de températures va de 190 à 210°C, la quantité de platine est d'au moins 0,0008 % en poids et la quantité d'hydroxyde de potassium ou de sodium est d'au moins 1,4 % en poids.

**10.** Procédé suivant la revendication 3 ou 4, dans lequel la température est d'environ 183°C, la quantité de platine est d'environ 0,0024 % en poids et la quantité d'hydroxyde de potassium ou d'hydroxyde de sodium est d'environ 5,1 % en poids.

**11.** Procédé suivant la revendication 10, dans lequel on utilise de l'hydroxyde de potassium.